# DEMANDE DE BREVET EUROPEEN

(11) **EP 3 103 432 A1**
(43) Date de publication de la demande: **14.12.2016**
(21) Numéro de dépôt: 16173162.5
(22) Date de dépôt: 06.06.2016
(51) Int. Cl.: A61K 8/25, A61K 8/34, A61Q 3/02, A61K 8/81, A61K 8/87

(54) **COMPOSITION COSMÉTIQUE AQUEUSE POUR VERNIS À ONGLES PRÉSENTANT UN EFFET SOFT TOUCH**

(30) Priorité: 11.06.2015 LU 92739
(71) Demandeur: International Lacquers S.A., 3225 Bettembourg (LU)
(72) Inventeur: POLIS, Aline, 57570 Beyren Les Sierck (FR); DEROSIER, Frédéric, 57680 Corny sur Moselle (FR); DELAS, Christophe, 3926 Mondercange (LU)
(74) Mandataire: Office Freylinger

(57) **Abrégé**

La présente invention concerne une composition cosmétique aqueuse pour vernis à ongles comprenant au moins un agent filmogène de type polyuréthane, une poudre silicone hybride, un agent de rhéologie et un agent conservateur. La composition selon l'invention permet de former une surface particulière de type soft touch caractérisée à la fois par un fini mat et une sensation caoutchouc, un toucher gomme agrippant et velouté.

## Description

### Domaine technique

La présente invention concerne une composition cosmétique qui peut s'appliquer soit directement sur les ongles ou les faux ongles, soit sur les ongles ou les faux ongles préalablement maquillés avec un vernis transparent appelé aussi base coat.

### Etat de la technique

Les vernis à ongles sont généralement utilisés pour maquiller les ongles ainsi que les faux ongles. La très grande majorité des vernis à ongles vendus sur le marché ont pour constituant principal des solvants organiques volatiles. Ces mêmes compositions comprennent également des agents filmogènes, des résines, un ou plusieurs agents de rhéologie ainsi que des colorants compatibles avec les solvants organiques volatiles mis en oeuvre tels que l'acétate d'éthyle ou l'acétate de butyle.

D'une manière générale, pour répondre aux exigences des consommateurs, les vernis à ongles doivent répondre à plusieurs critères :
- Une bonne application. Le vernis doit former une surface homogène et lisse, sans stries et sans bulles.
- Un étalement optimisé sur toute la surface de l'ongle.
- Une brillance accrue qui tient dans le temps.
- Un temps de séchage relativement court.
- Une bonne tenue dans le temps avec le minimum d'écaillement et le minimum d'usure à l'extrémité des ongles.

Afin de satisfaire à ses exigences, et en particulier améliorer ces différentes propriétés, de nouvelles formulations ou typologies de formules sont développées.

En addition de satisfaire à toutes les caractéristiques précédemment évoquées, les utilisateurs réclament en permanence des vernis à ongles présentant de nouveaux effets esthétiques. Le vernis à ongles est devenu un véritable accessoire de mode et doit s'adapter à l'évolution des tendances.

Les vernis doivent donc s'adapter aux tendances colorielles par la mise en oeuvre de matières colorantes spécifiques mais ils doivent aussi s'adapter à des tendances qui sont plus en relation avec son fini de surface. Les dernières années ont ainsi vu se commercialiser des vernis à ongles à effet miroir, craquelé, métallisé ou mat. Ainsi, la demande US201220294918 décrit un vernis avec un toucher particulier décrit comme « velvety feel ». La demande EP2420222 décrit une composition ayant un toucher velours et la demande WO2012022499 décrit un vernis à ongles ayant une haptique veloutée.

En particulier, l'effet soft touch présent dans de nombreux objets de la vie courante, se caractérise notamment par une sensation caoutchouc, un toucher gomme agrippant et velouté.

### Objet de l'invention

Un objet de la présente invention est une composition cosmétique pour vernis à ongles présentant un effet soft touch après séchage tout en maintenant ses autres propriétés.

### Description générale de l'invention

Afin de résoudre le problème mentionné ci-dessus, à savoir la formulation d'un vernis à effet soft touch, la présente invention propose une composition aqueuse ne contenant pas de solvants organiques volatiles. La composition selon l'invention comprend un ou plusieurs agents filmogènes, un ou plusieurs agents de rhéologie compatibles avec les agents filmogènes, un ou plusieurs agents conservateurs de la formule et une poudre silicone hybride apportant l'effet mat recherché ainsi que l'effet soft touch agrippant et velouté en combinaison avec l'un des filmogènes utilisés. De plus, les compositions selon l'invention ont la particularité de former un film dur et tenace et ne nécessitent pas de solvant organique volatile. La composition selon l'invention comprend de préférence aussi au moins un agent plastifiant, et peut être sans couleur ou peut comprendre au moins une matière colorante se présentant sous la forme de pigments organiques ou inorganiques, de nacres ou toute autre matière colorante connue de l'homme de l'art.

Il est à noter que dans le contexte de la présente invention le terme "ongle" désigne tant les ongles naturels que les faux ongles.

Conformément à l'invention, l'effet soft touch de la composition aqueuse est dû à la combinaison de la poudre silicone hybride et d'un ou de plusieurs agents filmogènes de type polyuréthane, de préférence de haut poids moléculaire dispersé dans l'eau.

La poudre silicone hybride (aussi appelée poudre siliconée) se présente généralement sous la forme de particules sphériques de diamètre moyen allant de 2 à 25 µm, de manière préférée de 4 à 15 µm. Ces particules sont généralement des élastomères organopolysiloxanes réticulés recouverts d'une résine silicone, en particulier une résine silsesquioxane comme décrit par exemple dans le brevet US 5,538,793. Ce type d'élastomère est commercialisé par la société Shin-Etsu sous les dénominations KSP-100, KSP-101, KSP-102, KSP-103, KSP-104, KSP-105 et KSP-300. Une poudre silicone hybride particulièrement avantageuse est de type vinyl dimethicone / methicone silsesquioxane crosspolymer, par exemple de type KSP-100 ou KSP-101. La poudre siliconée est utilisée dans le contexte de l'invention en général entre 2% et 20% en poids de la formule et de préférence entre 5% et 15% en poids.

Les polymères de type polyuréthane utilisables comme agents filmogènes préférés dans des compositions selon l'invention se présentent sous la forme de dispersions dans l'eau. Ces dispersions ont de préférence des taux de matière active de l'ordre de 30% à 50% en poids. Ces dispersions se présentent notamment sous la forme de liquides blancs et ont des viscosités inférieures à 1000 mPa.s. Ces dispersions seront de préférence choisies parmi les dispersions commercialisées sous les dénominations Baycusan C-1000 (INCl: Polyuréthane-34), Baycusan C-1001 (INCl: Polyuréthane-34), Baycusan C-1003 (INCl: Polyuréthane-32) par Bayer. La quantité de dispersion de polyuréthane présente dans la composition est comprise entre 5% et 45% de la composition et de préférence entre 15% et 40% en poids de la composition.

Le solvant principal de la composition selon l'invention est l'eau. La quantité d'eau présente dans la formule est généralement comprise entre 20% et 65% en poids.

Un co-solvant peut être utilisé dans la formule. Ces co-solvants sont des ingrédients volatiles solubles dans l'eau qui peuvent être utilisés notamment pour moduler le temps de séchage de la formule ou améliorer la solubilisation ou la dispersion des autres ingrédients présents dans la composition selon l'invention.

Parmi les co-solvants utilisables, on citera notamment les alcools de faible poids moléculaire comme l'éthanol et l'isopropanol.

Les co-solvants pourront également être choisis parmi les glycols comme le propylène glycol et le butylène glycol.

En variante ou en plus, les co-solvants pourront être aussi choisis parmi les éthers de glycol, on citera par exemple le 1,2-diméthoxyéthane ou le 1-méthoxy-2-(2-méthoxyéthoxy)éthane.

Ces co-solvants peuvent être présents dans la composition selon l'invention à des quantités à des quantités de 1% à 15% en poids de la composition.

La composition selon l'invention peut également contenir un second polymère qui se présente également sous la forme d'une dispersion aqueuse. Ces polymères doivent être compatibles avec le ou les agents filmogènes. Parmi ces dispersions polymériques on pourra notamment citer :
- Les copolymères d'acrylates comme le Covacryl E14 commercialisé par la société Sensient ou les Dermacryl C et Dermacryl AQF commercialisés par la société Akzo Nobel.
- Les copolymères de sels d'ammonium acrylates comme le Syntran KL219CG commercialisé par Interpolymer ou la dispersion Worlee Micromer C60/42 commercialisée par Worlee,
- Les copolymères d'ammonium méthacrylate, de styrène et d'acrylates. Parmi ces polymères on pourra citer le Syntran 5620CG commercialisé par Interpolymer ou le Joncryl 77 commercialisé par BASF,
- Les copolymères d'ammonium acrylate, de styrène et d'acrylates (Syntran 5760).

Les copolymères se présentant sous la forme de dispersions peuvent être présents dans la composition selon l'invention dans des quantités allant de 15% à 50% en poids et de préférence de 20% à 45% en poids.

La composition selon l'invention pourra également contenir des plastifiants. Ces ingrédients servent de préférence à moduler la dureté de la formule et en conséquence, en diminuer l'écaillement le cas échéant. Ces plastifiants sont généralement des composés non volatiles et compatibles avec les milieux dans lesquels ils sont ajoutés. Parmi les plastifiants utilisables selon l'invention, on citera notamment :
- L'acétyl tributyl citrate,
- L'acétyle triéthyl citrate,
- Le triéthylcitrate,
- La glycérine.

Les plastifiants pourront être utilisés dans la composition selon l'invention dans des quantités allant de 0.5 à 10%, et de préférence entre 1% et 8% en poids de la composition.

La composition selon l'invention contient un ou plusieurs agents de rhéologie. Ces agents de rhéologie permettent de modifier la viscosité et la rhéologie de la formulation afin de :
- Moduler la viscosité de la composition,
- Optimiser l'application de la formulation avec un pinceau. L'application est un paramètre essentiel pour ce type de composition cosmétique. L'application avec un pinceau doit permettre de former un film homogène en s'étalant de manière optimum sur l'ongle directement ou sur l'ongle préalablement recouvert d'un base coat. Le paramètre d'étalement de la composition sur le support est capital pour l'utilisateur.
- Permettre la tenue en suspension des particules solides au sein de la formulation. Ces agents évitent notamment la sédimentation des particules siliconées ou encore des agents colorants mis en oeuvre.

Parmi les agents de rhéologie utilisables dans la composition selon l'invention, on citera notamment :
- Les argiles compatibles avec les milieux aqueux comme la bentone EW (nom INCl: Hectorite).
- Les dérivés de cellulose comme l'hydroxyéthylcellulose commercialisé sous la dénomination Natrosol par Ashland.
- Les polyacrylates commercialisés sous la dénomination Sepiplus 400. Cet ingrédient est un mélange constitué de trois ingrédients (Polyacrylate-13, Polyisobutene et Polysorbate 20).
- Les polyacrylamides commercialisés sous la dénomination Sepigel 305 par Seppic. Le sepigel 305 se présente sous la forme d'un mélange constitué de trois ingrédients : Polyacrylamide, C13-14 Isoparaffin et Laureth-7.
- Les silicates. On citera notamment la Laponite XLS (INCl: Lithium Magnesium Sodium Silicate & tetrasodium pyrophosphate) commercialisée par Byk.
- Les silylates. On citera notamment l'ingrédient Aerosil R972 (INCl: silica diméthyl silylate) commercialisé par Degussa.

Selon un mode de réalisation de l'invention, la composition pour effet soft touch comprend une quantité d'agent(s) de rhéologie comprise entre 0.1% et 3.5% en poids, et de préférence entre 0.5% et 2% en poids.

Compte tenu de sa nature aqueuse, la composition selon l'invention doit contenir un ou plusieurs agents conservateurs. Ces conservateurs sont nécessaires pour empêcher le développement de toute contamination bactérienne ou le développement de moisissures. Les conservateurs susceptibles d'être utilisés sont par exemple :
- L'alcool benzylique (CAS 100-51-6),
- Le phenoxyéthanol (CAS 122-99-6) utilisé seul ou en combinaison avec l'éthylhexylglycérine (CAS 70445-33-9),
- Le formaldéhyde (CAS 50-00-0),
- Le dihydroacétate de sodium (CAS 4418-26-2),
- Le tétrasodium EDTA (CAS 64-02-8),
- Le méthylchloroisothiazolinone (CAS 26172-55-4),
- L'acide sorbique et ses sels (CAS 110-44-1, 7492-55-9, 7757-81-5, 24634-61-5),
- Le méthylisothiazolinone (CAS 2682-20-4),
- Le méthylparaben (CAS 99-76-3),
- Le propylparaben (CAS 94-13-3),
- Le diazolidinylurea (CAS 78491-02-8),
- La chlorphénésine (CAS 104-29-0)
- L'acide benzoïque et son sel de sodium (CAS 65-85-0 et 532-35-1).

Les conservateurs seront utilisés à des pourcentages connus par l'homme de l'art qui tiendra compte également des réglementations en vigueur qui imposent généralement des pourcentages maximum d'utilisation dans les compositions cosmétiques.

La composition selon l'invention peut ne pas contenir de matière colorante ; elle se présente alors sous la forme d'un liquide blanchâtre qui devient transparente au séchage. Seul le fini soft touch est présent. Ou la composition comprendra un ou plusieurs agents de coloration. On entend par agent de coloration toute matière première susceptible de modifier la couleur de ladite composition. D'une manière non limitative on citera comme agent de coloration les pigments, les nacres, les paillettes et les particules métalliques.

Les pigments utilisables dans la composition selon l'invention, pourront être sélectionnés parmi le groupe constitué par le dioxyde de titane (CI 77891), l'oxyde de fer noir (CI 77499), l'oxyde de fer rouge (CI 77491), le DC Red 6 Ba Lake (CI 15850), le DC Red 7 Lake (CI 15850 :1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le Carmin (CI 75470), les oxydes de chrome (CI 77288 et CI 77289), le Bleu de Prusse (CI 77510). Les pigments fluorescents, phosphorescents, thermochromiques et photochromiques pourront également être utilisés dans la composition selon l'invention.

Les nacres pourront être mises en oeuvre pour apporter de la couleur ou des effets à la composition selon l'invention. Les nacres sont généralement décrites comme des particules minérales naturelles ou synthétiques (mica naturel, mica synthétique appelé également fluorphlogopite, calcium sodium borosilicate, aluminium calcium sodium silicate) recouvertes d'une ou plusieurs couches d'oxydes métalliques, de métaux ou de pigments. Les nacres se présentent sous la forme de poudres ou de particules de différentes tailles allant de 5 µm à 750 µm dans leur dimension la plus grande.

La composition selon l'invention pourra comprendre également des paillettes. Les paillettes sont des particules plastiques de faible taille constituées par exemple de couches de polyéthylène téréphtalate ou de polyuréthane prenant en sandwich des pigments. Les paillettes peuvent avoir des tailles et des formes variables. Ainsi les paillettes peuvent être rondes, hexagonales, carrées, en forme de bâtonnet. Il est important de noter que les paillettes peuvent également prendre la forme de tout signe, logo ou design particulier.

La composition selon l'invention comprendra éventuellement des particules métalliques. En particulier la composition pourra inclure des particules d'aluminium de taille et de forme variable selon l'effet recherché.

Selon un mode de réalisation de l'invention, la composition selon l'invention comprend une quantité d'agent de coloration comprise entre 0.1% et 20% en poids total de la composition. De préférence, la quantité et la nature des agents de coloration utilisés dans la composition selon l'invention seront choisis de manière à ce qu'ils n'interfèrent pas avec l'effet soft touch revendiqué.

La composition aqueuse pour vernis à ongles peut en outre comprendre différents additifs choisis parmi les absorbeurs UV et les agents dits « traitants ».

Les différents additifs utilisables dans l'invention sont :

Parmi les absorbants UV, on citera les absorbants UV qui permettent de protéger la composition ou les agents de coloration utilisés selon l'invention des rayonnements UV. Parmi les protecteurs UV utilisables dans l'invention, on citera la benzophénone-1 (CAS 131-56-6), la benzophénone-3 (CAS 131-57-7), la benzophénone-5 (CAS 6628-37-1), l'étocrylène (CAS 5232-99-5).

Les absorbants UV seront utilisés si nécessaire, seuls ou sous la forme de combinaisons dont le pourcentage total dans la formule peut aller de 0.1 à 1 % en poids total de la formulation.

On citera également les additifs traitants de l'ongle utilisables dans la composition selon l'invention. Les additifs traitants peuvent représenter de 0.01% à 5% en poids total de la composition. Parmi ces additifs dits « traitants », on citera sans limitation les ingrédients suivants :
- Le formaldéhyde (CAS 50-00-0),
- La vitamine E acétate (CAS 58-95-7),
- La vitamine A palmitate (CAS 79-81-2),
- Les dérivés organiques ou inorganiques de calcium tels que le chlorure de calcium (CAS 10043-52-4), le pantothénate de calcium (CAS 137-08-6),
- Les acides aminés soufrés comme la cystéine (52-90-4), ses sels et leurs dérivés et la cystine (CAS 56-89-3),
- La kératine hydrolysée d'origine végétale,
- Les alpha-hydroxyacides comme l'acide citrique (CAS 77-92-9), l'acide malique (CAS 617-48-1) ou l'acide ascorbique (CAS 50-81-7),
- La biotine (58-85-5),
- L'urée (CAS 57-13-6).

L'invention concerne également un procédé de préparation de la composition aqueuse pour vernis à ongles. Ce procédé de préparation comprend l'étape de mélange de un ou plusieurs filmogènes, d'une poudre silicone hybride, d'un ou plusieurs agents de conservation et d'un ou plusieurs agents rhéologiques, de préférence en y intégrant d'autres composants comme décrit ci-dessus.

De préférence, la viscosité de la formulation aqueuse selon l'invention est comprise entre 100 mPa.s et 300 mPa.s, viscosité mesurée à 60 RPM avec un viscosimètre de type Brookfield.

### Exemples

Les exemples ci-dessous illustrent des formulations aqueuses possibles pour vernis à ongles présentant un effet soft touch.

### Exemples 1 à 4 :

Les ingrédients "silicone" suivants ont été testés, les trois premiers étant des composés de poudre silicone hybride et la diméthicone étant une silicone ne correspondant pas à cette définition :
- KSP-100 : vinyl dimethicone / methicone silsesquioxane crosspolymer, diamètre moyen des particules environ 5 µm
- KSP-101 : vinyl dimethicone / methicone silsesquioxane crosspolymer, diamètre moyen des particules environ 12 µm
- KSP-300 : Diphenyl dimethicone / Vinyl diphenyl dimethicone / Silsesquioxane Crosspolymer, diamètre moyen des particules environ 5 µm
- Dow Corning 9701: dimethicone / vinyl dimethicone crosspolymer and Silica
- Diméthicone

| Eau déminéralisée | qsp 100.00 % | qsp 100.00 % | qsp 100.00% | qsp 100.00% |
|---|---|---|---|---|
| Baycusan C 1001 | 33.50 % | 33.50 % | 33.50 % | 33.50 % |
| Joncryl 77 | 29.50 % | 29.50 % | 29.50 % | 29.50 % |
| KSP-101 | 10.30 % | | | |
| KSP-100 | | 10.30 % | | |
| KSP-300 | | | 10.30 % | |
| Dow Corning 9701 | | | | 10.30 % |
| Propylene Glycol | 5.80 % | 5.80 % | 5.80 % | 5.80 % |
| Sepiplus 400 | 0.93% | 0.93% | 0.93% | 0.93% |
| Sorbate de | 0.20 % | 0.20 % | 0.20 % | 0.20 % |
| potassium | | | | |
| Ethylhexylglycerin | 0.1 % | 0.1 % | 0.1 % | 0.1 % |
| Phenoxyethanol | 0.9% | 0.9% | 0.9% | 0.9% |
| DC Red 6 Ba Lake | 0.2 % | 0.2 % | 0.2 % | 0.2 % |

L'effet soft touch avec KSP-100 et KSP-101 est très marqué. Celui avec KSP-300 et Dow Coming 9701 est notable, mais pas aussi marqué qu'avec KSP-100. Avec la diméthicone (pas selon l'invention), l'effet est absent.

## Revendications

1. Composition aqueuse pour vernis à ongles présentant un effet soft touch après application sur les ongles, comprenant au moins un agent filmogène de type polyuréthane, une poudre silicone hybride, un agent conservateur et un agent de rhéologie.

2. Composition aqueuse pour vernis à ongles selon la revendication 1, dans laquelle la poudre silicone hybride comprend des particules sphériques d'élastomères organopolysiloxanes réticulés recouverts d'une résine silicone.

3. Composition aqueuse pour vernis à ongles selon la revendication 2, dans laquelle la résine silicone est une résine silsesquioxane.

4. Composition aqueuse pour vernis à ongles selon l'une quelconque des revendications 1 à 3, dans laquelle la poudre silicone hybride est du type vinyl dimethicone / methicone silsesquioxane crosspolymer.

5. Composition aqueuse pour vernis à ongles selon l'une quelconque des revendications 1 à 4, dans laquelle la quantité de poudre silicone hybride est comprise entre 2% et 20% en poids, de préférence entre 5% et 15% en poids de la composition totale.

6. Composition aqueuse pour vernis à ongles selon l'une quelconque des revendications 1 à 5, comprenant en outre au moins une matière colorante.

7. Composition aqueuse pour vernis à ongles selon l'une quelconque des revendications 1 à 6, dans laquelle la quantité d'agent(s) filmogène(s) de type polyuréthane est comprise entre 5% et 45% en poids, de préférence entre 15% et 40% en poids de ladite composition.

8. Composition aqueuse pour vernis à ongles selon l'une quelconque des revendications 1 à 7, dont la viscosité est comprise entre 100 mPa.s et 300 mPa.s, viscosité mesurée à 60 RPM avec un viscosimètre de type Brookfield.

9. Procédé de préparation d'une composition aqueuse pour vernis à ongles présentant un effet soft touch après application sur les ongles, le procédé comprenant l'étape de mélange d'un ou plusieurs agents filmogènes de type polyuréthane, d'une poudre silicone hybride, d'un ou plusieurs agents conservateurs et d'un ou plusieurs agents de rhéologie.
